# EUROPÄISCHE PATENTSCHRIFT

(11) **EP 4 072 468 B1**
(45) Veröffentlichungstag und Bekanntmachung des Hinweises auf die Patenterteilung: **26.03.2025**
(21) Anmeldenummer: 20824468.1
(22) Anmeldetag: 04.12.2020
(51) Int. Cl.: A61F 2/16

(54) **AUGENIMPLANTAT FÜR EINE AKKOMMODIERENDE INTRAOKULARLINSE**
EYE IMPLANT FOR AN ACCOMMODATIVE INTRAOCULAR LENS
IMPLANT OCULAIRE POUR LENTILLE INTRAOCULAIRE ACCOMMODATIVE

(30) Priorität: 12.12.2019 DE 102019134169
(43) Veröffentlichungstag der Anmeldung: 19.10.2022
(73) Patentinhaber: Carl Zeiss Meditec AG, 07745 Jena (DE)
(72) Erfinder: BUCHHEISTER, Jan, 07751 Jena (DE); TROEGER, Stefan, 08328 Stützengrün (DE); WOLFSTEIN, André, 13507 Berlin (DE); SRBINOSKA, Hristina, 14532 Kleinmachnow (DE); CZAP, Almut, 73432 Aalen (DE)
(74) Vertreter: PATERIS Patentanwälte PartmbB
(86) Internationale Anmeldenummer: PCT/EP2020/084692
(87) Internationale Veröffentlichungsnummer: WO 2021/115964

(56) Entgegenhaltungen:
- US-A1- 2005 251 253
- US-A1- 2011 029 074
- US-B2- 10 327 889

## Beschreibung

Die Erfindung betrifft ein Augenimplantat für eine akkommodierende Intraokularlinse.

Unter Akkommodation wird die Fähigkeit des Auges verstanden, die Brechkraft dynamisch anzupassen, und dadurch Gegenstände in unterschiedlicher Entfernung scharf zu sehen. Dabei wird eine Durchmesseränderung eines Ziliarmuskels des Auges über Zonulafasern auf einen flexiblen Kapselsack übertragen, in dem eine flexible Linse angeordnet ist. Allerdings verschlechtert sich mit zunehmenden Alter die Fähigkeit zu der Akkommodation, was zur Folge eine Altersweitsichtigkeit hat. Dies liegt vor allem in einer geringer werdenden Elastizität der Linse, die sich mit dem zunehmenden Alter verfestigt. Mittels einer akkommodierenden Intraokularlinse, die von dem Ziliarmuskel verformbar ist, wird versucht, die Fähigkeit des Auges zur Akkommodation beizubehalten. Alle derzeit bekannten akkommodierenden Intraokularlinsen ermöglichen jedoch keine ausreichende Kraftübertragung von dem Ziliarmuskel auf die akkommodierende Intraokularlinse, um die Akkommodation zu ermöglichen.

US 2005/0251253 A1 offenbart eine Intraokularlinsenanordnung. US 2011/0029074 A1 offenbart eine ophthalmische Vorrichtung zum Implantieren in den Kapselsack eines Auges. US 10,327,889 B2 offenbart ein Spannungsmittel zum Befestigen an einer hinteren Kapsel eines Auges.

Aufgabe der Erfindung ist es daher, ein Augenimplantat zu schaffen, mit dem eine Kraftübertragung von einem Ziliarmuskel auf eine akkommodierende Intraokularlinse gut funktioniert.

Das erste erfindungsgemäße Augenimplantat weist eine Linsenhülle, die eingerichtet ist, in einen Kapselsack eines Auges eingesetzt zu werden, elastisch ist, in ihrem Inneren einen Hohlraum begrenzt und ein Durchgangsloch aufweist, via das von außerhalb der Linsenhülle der Hohlraum zugänglich ist, eine Mehrzahl an Reservoiren, die an der Linsenhülle befestigt sind, von der Linsenhülle nach außen abstehen, in ihrem Inneren einen Klebstoff aufweisen und eingerichtet sind, wenn sie auf den Kapselsack gedrückt werden, den Klebstoff abzugeben, und mindestens zwei Adapter auf, die an der Linsenhülle befestigt sind, von der Linsenhülle nach innen abstehen und eingerichtet sind, in Eingriff mit jeweils einer Haptik einer akkommodierenden Intraokularlinse zu stehen. Die Linsenhülle kann Silikon und/oder Polyurethan aufweisen oder daraus bestehen. Das Material der Linsenhülle oder der Reservoire kann ein Formgedächtnismaterial aufweisen. Die Reservoire können Silikon und/oder Polyurethan aufweisen oder daraus bestehen. Bei den Reservoiren kann es sich beispielsweise um Blasen handeln.

Bei dem Klebstoff kann es sich beispielsweise um einen "Superklebstoff" handeln, wie er unter https://www.berlin-university-alliance.de/impressions/20180110-mussel-basedsuper-adhesive/index.html beschrieben ist. Alternativ kann es sich bei dem Klebstoff um sogenannte "tissue adhesives" oder um HLAA (hydrophobic light-activated adhesive) der Firma Geck Biomedical, Paris, vgl. https://www.deutsche-apothekerzeitung.de/daz-az/2014/daz-3-2014/chirurgischer-klebstoff, handeln. Auch ist denkbar, dass es sich bei dem Klebstoff um einen Zwei-Komponenten-Klebstoff handelt. Die zwei Komponenten des Zwei-Komponenten-Klebstoffs können in zwei getrennten Kammern in dem Reservoir angeordnet sein und sich vermischen, wenn die Reservoire auf den Kapselsack gedrückt werden. Der Klebstoff kann zudem elastisch sein.

Das zweite erfindungsgemäße Augenimplantat weist eine Linsenhülle, die eingerichtet ist, in einen Kapselsack eines Auges eingesetzt zu werden, elastisch ist, in ihrem Inneren einen Hohlraum begrenzt und ein Durchgangsloch aufweist, via das von außerhalb der Linsenhülle der Hohlraum zugänglich ist, eine Mehrzahl an Schutzhüllen, die an der Linsenhülle befestigt sind und von der Linsenhülle nach außen abstehen, jeweils einen Widerhaken für jede Schutzhülle, der in dem Inneren der zugehörigen Schutzhülle eingekapselt ist, mit der Linsenhülle verbunden ist, von der Linsenhülle nach außen absteht und eingerichtet ist, wenn der Widerhaken auf den Kapselsack gedrückt wird, die zugehörige Schutzhülle zu durchstoßen und in dem Kapselsack zu verhaken, und mindestens zwei Adapter auf, die an der Linsenhülle befestigt sind, von der Linsenhülle nach innen abstehen und eingerichtet sind, in Eingriff mit jeweils einer Haptik einer akkommodierenden Intraokularlinse zu stehen.

Das Augenimplantat ist dazu vorgesehen, in den Kapselsack des Auges eingesetzt zu werden, nachdem die natürliche Linse des Auges von einem Operateur mittels Phakoemulsifikation zerkleinert wurde und abgesaugt wurde. Das Augenimplantat wird in den Kapselsack eingesetzt, wenn der Hohlraum der Linsenhülle möglichst leer ist und somit die Linsenhülle einen geringen Platzbedarf hat. Anschließend wird die Linsenhülle aufgepumpt, indem mittels einer Kanüle eine Operationsflüssigkeit via das Durchgangsloch in den Hohlraum gedrückt wird. Dazu ist es denkbar, dass das Durchgangsloch eingerichtet ist, die Kanüle abzudichten, damit wenig oder nichts von der Operationsflüssigkeit aus dem Hohlraum gelangen kann, während die Linsenhülle aufgepumpt wird. Indem die Linsenhülle flexibel ist, kann sie sich an verschieden große der Kapselsäcke anpassen. Mittels des Klebstoffs oder des Widerhakens wird das Augenimplantat an dem Kapselsack befestigt. Durch das Vorsehen der Reservoire gemäß dem ersten erfindungsgemäßen Augenimplantat ist der Klebstoff während des Einsetzens des Augenimplantats in den Kapselsack innerhalb der Reservoire gehalten, wodurch es möglich ist, das Augenimplantat zu komprimieren und/oder zu falten, ohne dass der Klebstoff das Augenimplantat verklebt. Indem das Augenimplantat komprimiert und/oder gefaltet wird, kann das Augenimplantat via einen nur kleinen Schnitt in der Hornhaut in den Kapselsack eingebracht werden. Beispielsweise ist denkbar, das Augenimplantat via den gleichen Schnitt in der Hornhaut des Auges einzubringen, via den auch die natürliche Linse abgesaugt wurde. Analog kann durch das Vorsehen der Schutzhüllen gemäß des zweiten erfindungsgemäßen Augenimplantats das Augenimplantat komprimiert und/oder gefaltet werden, ohne dass dabei die Widerhaken das Augenimplantat beschädigen. Nachdem die Linsenhülle aufgepumpt wurde, wird von dem Operateur eine Einsetzöffnung in die Linsenhülle eingebracht, beispielsweise mittels eines Messers oder einer Pinzette. Anschließend wird die akkommodierende Intraokularlinse via die Einsetzöffnung in die Linsenhülle eingesetzt und die Haptiken werden von dem Operateur mit jeweils einem der Adapter in Eingriff gebracht, wodurch die akkommodierende Intraokularlinse an dem Augenimplantat befestigt wird. Weil das Augenimplantat an dem Kapselsack befestigt ist, erfolgt eine gute Kraftübertragung von einem Ziliarmuskel des Auges auf das Augenimplantat. Weil die akkommodierende Intraokularlinse an dem Augenimplantat befestigt ist, ist eine Kraftübertragung von dem Augenimplantat auf die akkommodierende Intraokularlinse gut, wodurch insgesamt eine Kraftübertragung von dem Ziliarmuskel auf die akkommodierende Intraokularlinse gut ist. Das Augenimplantat kann dabei an dem Kapselsack befestigt werden, während die Linsenhülle aufgepumpt wird und/oder während die akkommodierende Intraokularlinse in die Linsenhülle eingesetzt wird.

Für die erste erfindungsgemäße Ausführungsform ist es bevorzugt, dass die Reservoire jeweils eine Sollbruchstelle haben, die eingerichtet ist, wenn die Reservoire auf den Kapselsack gedrückt werden, geöffnet zu werden, oder wobei die Reservoire jeweils eine Reservoiröffnung aufweisen, die eingerichtet ist, den Klebstoff passieren zu lassen, wenn die Reservoire auf den Kapselsack gedrückt werden.

Für die erste erfindungsgemäße Ausführungsform ist es bevorzugt, dass die Linsenhülle für jede der Reservoire eine Hüllenöffnung aufweist und an jeder der Hüllenöffnungen einer der Adapter angeordnet ist, der den Klebstoff kontaktiert. Wenn die Haptiken mit dem jeweils einen der Adapter in Eingriff stehen, drückt der Adapter den Klebstoff auf den Kapselsack. Dadurch kann das Augenimplantat vorteilhaft besonders fest an dem Kapselsack befestigt werden.

Für die zweite erfindungsgemäße Ausführungsform ist es bevorzugt, dass jeder der Widerhaken sich durch die Linsenhülle erstreckt und an einem der Adapter befestigt ist. Wenn die Haptiken mit dem jeweils einen der Adapter in Eingriff stehen, drückt der Adapter den Widerhaken auf den Kapselsack. Dadurch kann das Augenimplantat vorteilhaft besonders fest an dem Kapselsack befestigt werden. Zudem ist der Widerhaken mittels des Adapters besonders fest befestigt, insbesondere im Vergleich dazu, wenn der Widerhaken lediglich an der Linsenhülle befestigt wäre.

Für die zweite erfindungsgemäße Ausführungsform ist es bevorzugt, dass die Schutzhüllen eingerichtet sind, sich nach Einsetzen des Augenimplantats in den Kapselsack zu zersetzen.

Für beide erfindungsgemäßen Ausführungsformen ist es bevorzugt, dass die Linsenhülle diskusförmig ist. Diese Form der Linsenhülle lässt sich vorteilhaft einfach herstellen. Alternativ ist es für die beiden erfindungsgemäßen Ausführungsformen bevorzugt, dass die Linsenhülle torusförmig ist. Dadurch kann die Linsenhülle vorteilhaft platzsparend ausgeführt werden. Dies ist insbesondere relevant, wenn sie komprimiert und/oder gefaltet wird, um in den Kapselsack eingesetzt zu werden. Zudem ist es möglich, die torusförmige Ausführungsform der Linsenhülle so in den Strahlgang des Auges zu positionieren, dass wenig oder kein Licht auf die Netzhaut trifft, das vorher die Linsenhülle durchstrahlt hat.

Es ist für beide erfindungsgemäßen Ausführungsformen bevorzugt, dass die Linsenhülle eine Linsenhüllenverdickung aufweist, die um das Durchgangsloch herum angeordnet ist. Dadurch kann eine Gefahr einer Beschädigung der Linsenhülle, während sie aufgepumpt wird, vermindert werden. Besonders bevorzugt ist die Linsenhüllenverdickung außen an der Linsenhülle angeordnet. Dadurch ist es möglich, die Kanüle, während die Operationsflüssigkeit in den Hohlraum gedrückt wird, positionsstabil innerhalb der Linsenhüllenverdickung zu halten.

Für beide Ausführungsformen ist es bevorzugt, dass die Linsenhülle ein Ventil aufweist, das das Durchgangsloch aufweist. In dem Fall, dass die Linsenhüllenverdickung vorgesehen ist, kann die Linsenhüllenverdickung um das Ventil herum angeordnet sein oder ein Teil des Ventils sein.

Die Linsenhülle weist für beide Ausführungsformen bevorzugt eine Hüllensollbruchstelle auf. Durch ein Öffnen der Hüllensollbruchstelle kann die Einsetzöffnung besonders einfach hergestellt werden. Beispielsweise kann es sich bei der Hüllensollbruchstelle um eine Perforierung handeln. In dem Fall, dass die Linsenhüllenverdickung vorgesehen ist, kann die Hüllensollbruchstelle um die Linsenhüllenverdickung angeordnet sein. Dadurch kann zum Herstellen der Einsetzöffnung die Linsenhüllenverdickung mittels einer Pinzette gegriffen werden.

Besonders bevorzugt sind zwei der Hüllensollbruchstellen vorgesehen, die gegenüberliegend an der Linsenhülle vorgesehen sind. Dadurch kann die Einsetzöffnung unabhängig davon hergestellt werden, wie das Augenimplantat in dem Kapselsack angeordnet ist.

Für beide Ausführungsformen ist es bevorzugt, dass jeder der Adapter einen ersten Adapterarm und einen zweiten Adapterarm aufweist, die eingerichtet sind, dass zwischen ihnen die Haptik in Eingriff steht. Mittels des ersten Adapterarms und des zweiten Adapterarms ist es möglich, den Kapselsack aufzuspannen. Insbesondere ist es dadurch möglich zu unterbinden, dass der vordere Bereich des Kapselsacks und der hintere Bereich des Kapselsacks sich berühren. Durch das Aufspannen des Kapselsacks kann die Wahrscheinlichkeit für das Auftreten eines Nachstars und die Intensität des Nachstars vermindert werden. Indem der Kapselsack aufgespannt wird, werden die Transformations- und Wachstumsfaktoren, die von den äquatorialen Zellen abgegeben werden, durch zirkulierendes Kammerwasser verdünnt. Von den äquatorialen Zellen wandeln sich dann weniger in Fibroblasten um, wodurch auch eine Fibrose vermindert wird.

Es ist für beide Ausführungsformen bevorzugt, dass das Augenimplantat mindestens zwei Magneten oder magnetisierbare Augenimplantatsbereiche aufweist, die eingerichtet sind, jeweils eine der Haptiken an dem Augenimplantat zu befestigen. Dadurch kann die Intraokularlinse besonders fest an dem Augenimplantat befestigt werden. Die Magneten oder die magnetisierbaren Augenimplantatsbereiche können in der Linsenhülle und/oder in den Adaptern angeordnet sein. In dem Fall, dass die Magneten vorgesehen sind, können die Haptiken magnetisierbare Haptikbereiche oder Magneten aufweisen. In dem Fall, dass die magnetisierbaren Augenimplantatsbereiche vorgesehen sind, können die Haptiken Magnete aufweisen. Es ist denkbar, dass die Magnete in dem Augenimplantat und/oder in den Haptiken in Form von magnetischen Nanopartikeln vorliegen.

Bei beiden Ausführungsformen weist die Linsenhülle bevorzugt an ihrer Außenseite eine Skala auf. Dadurch ist erkennbar, wie weit die Linsenhülle aufgepumpt ist.

Bei beiden Ausführungsformen sind die Adapter bevorzugt so konfiguriert, dass die Intraokularlinse immer den gleichen radialen Durchmesser haben kann unabhängig davon, wie groß der Kapselsack ist.

Bei beiden Ausführungsformen weist die Operationsflüssigkeit bevorzugt ein Gleitmittel und/oder eine physiologische Salzlösung auf oder besteht aus dem Gleitmittel und/oder der physiologischen Salzlösung. Bei dem Gleitmittel kann es sich um ein sogenanntes opthalmisches Viskoelastikum handeln (engl.: opthalmic viscoelastic device (OVD)).

Ein erfindungsgemäßes Kit weist das Augenimplantat und die akkommodierende Intraokularlinse auf.

Im Folgenden wird anhand der beigefügten schematischen Zeichnungen die Erfindung näher erläutert.
Figur 1 zeigt einen Schnitt durch ein Auge mit einer ersten erfindungsgemäßen Ausführungsform eines Augenimplantats zu drei verschiedenen Zeitpunkten, wobei links der erste Zeitpunkt, in der Mitte der zweite Zeitpunkt und rechts der dritte Zeitpunkt dargestellt sind, wobei der Schnitt zu dem dritten Zeitpunkt senkrecht auf den Schnitten zu dem ersten Zeitpunkt und dem zweiten Zeitpunkt angeordnet ist.
Figur 2 zeigt einen Schnitt durch ein Auge mit einer zweiten erfindungsgemäßen Ausführungsform des Augenimplantats zu drei verschiedenen Zeitpunkten, wobei links der erste Zeitpunkt, in der Mitte der zweite Zeitpunkt und rechts der dritte Zeitpunkt dargestellt sind, wobei der Schnitt zu dem dritten Zeitpunkt senkrecht auf den Schnitten zu dem ersten Zeitpunkt und dem zweiten Zeitpunkt angeordnet ist.
Figur 3 zeigt eine Draufsicht auf zwei verschiedene Ausführungsformen einer Linsenhülle des Augenimplantats, wobei die Linsenhülle eine Linsenhüllenverdickung aufweist.
Figur 4 zeigt eine Draufsicht auf zwei verschiedene Ausführungsformen einer Linsenhülle des Augenimplantats, wobei die Linsenhülle eine Hüllensollbruchstelle aufweist.
Figur 5 zeigt einen Schnitt durch ein Auge mit einer Ausführungsform des Augenimplantats, bei der ein Adapter des Augenimplantats einen ersten Adapterarm und einen zweiten Adapterarm aufweist.

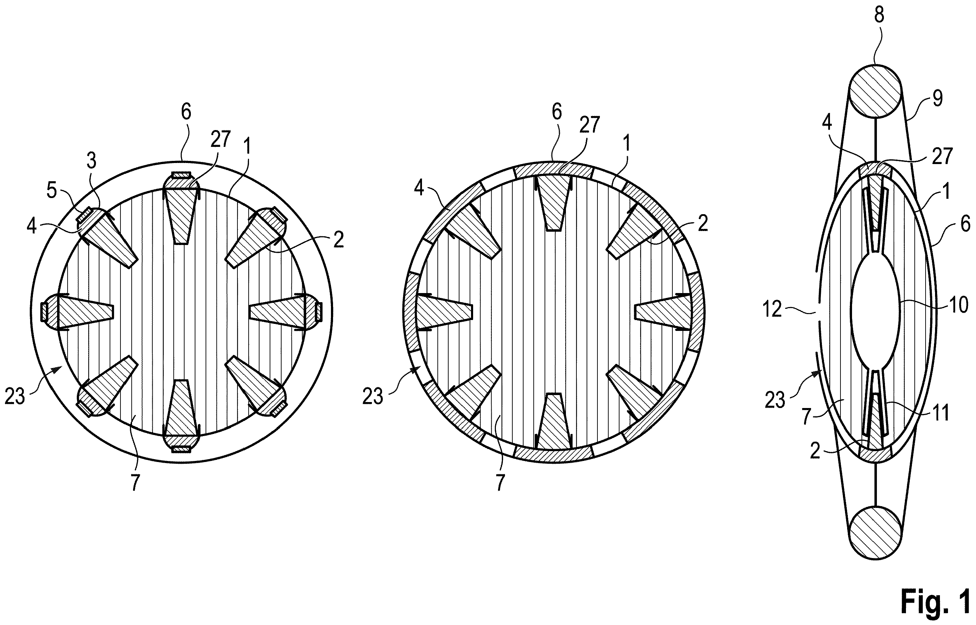

Wie es aus Figur 1 ersichtlich ist, weist ein Augenimplantat 23 gemäß einer ersten Ausführungsform eine Linsenhülle 1, eine Mehrzahl an Reservoiren 3 und mindestens zwei Adapter 2 auf.

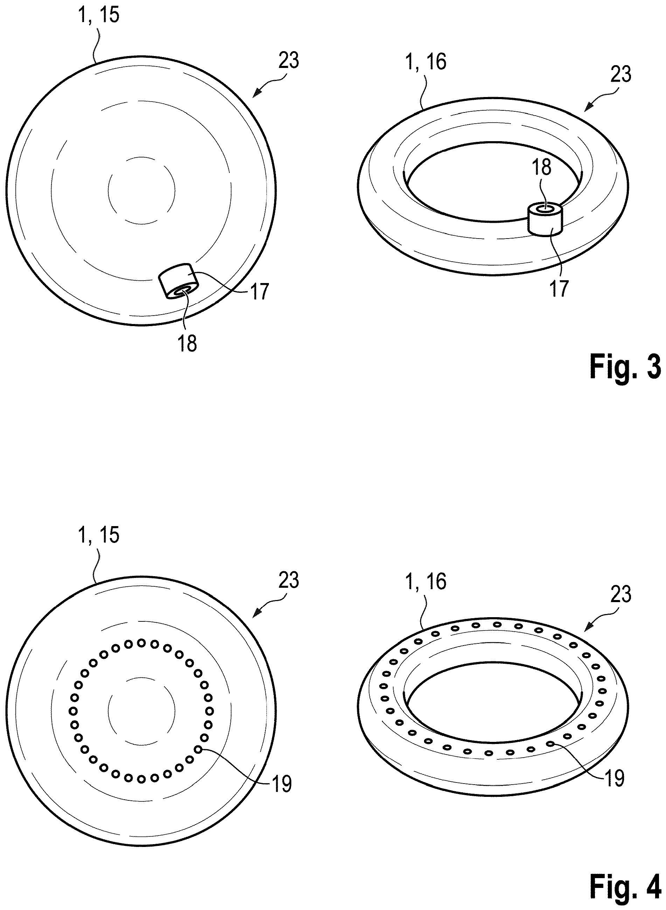

Die Linsenhülle 1 ist eingerichtet, in einen Kapselsack 6 eines Auges eingesetzt zu werden. Zudem begrenzt die Linsenhülle 1 in ihrem Inneren einen Hohlraum und weist ein Durchgangsloch 18 (nur in Figur 3 dargestellt) auf, via das von außerhalb der Linsenhülle 1 der Hohlraum zugänglich ist. Außerdem ist die Linsenhülle 1 elastisch. Die Reservoire 3 sind an der Linsenhülle 1 befestigt und stehen von der Linsenhülle 1 nach außen ab. Zudem weisen die Reservoire 3 in ihrem Inneren einen Klebstoff 4 auf und sind eingerichtet, wenn sie auf den Kapselsack 6 gedrückt werden, den Klebstoff 4 abzugeben. Die Adapter 2 sind an der Linsenhülle 1 befestigt und stehen von der Linsenhülle 1 nach innen ab. Zudem sind die Adapter 2 eingerichtet, in Eingriff mit jeweils einer Haptik 11 einer akkommodierenden Intraokularlinse 10 zu stehen.

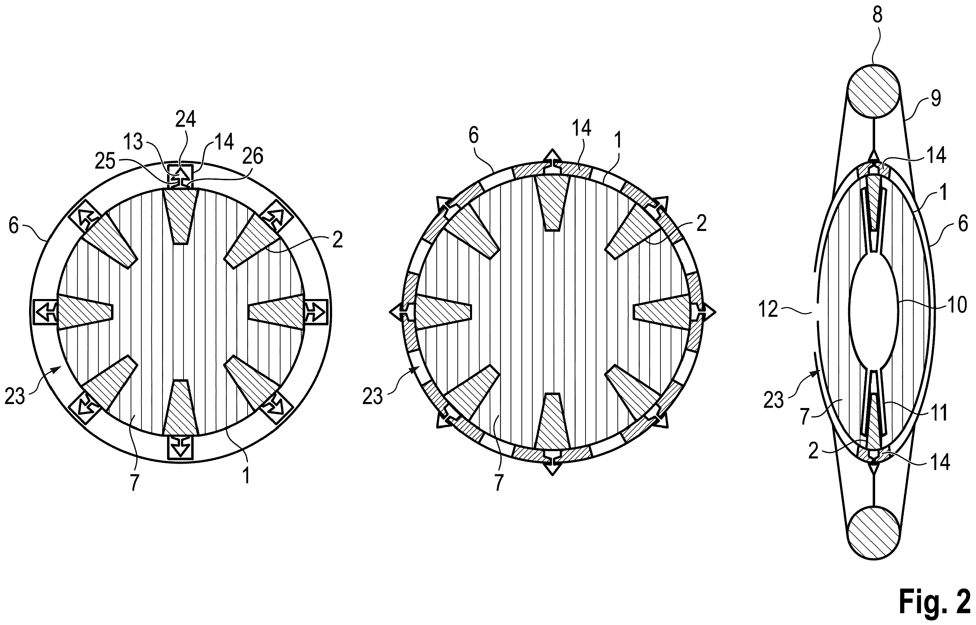

Wie es aus Figur 2 ersichtlich ist, weist ein Augenimplantat 23 gemäß einer zweiten Ausführungsform eine Linsenhülle 1, eine Mehrzahl an Schutzhüllen 14, jeweils einen Widerhaken 13 für jede der Schutzhüllen 14 und mindestens zwei Adapter 2 auf. Die Linsenhülle 1 ist eingerichtet, in einen Kapselsack 6 eines Auges eingesetzt zu werden. Zudem begrenzt die Linsenhülle 1 in ihrem Inneren einen Hohlraum und weist ein Durchgangsloch 18 auf, via das von außerhalb der Linsenhülle 1 der Hohlraum zugänglich ist. Außerdem ist die Linsenhülle 1 elastisch. Die Schutzhüllen 14 sind an der Linsenhülle 1 befestigt und stehen von der Linsenhülle 1 nach außen ab. Jeder Widerhaken 13 ist in dem Inneren der zugehörigen Schutzhülle 14 eingekapselt, mit der Linsenhülle 1 verbunden und steht von der Linsenhülle 1 nach außen ab. Zudem ist jeder Widerhaken 13 eingerichtet, wenn der Widerhaken 13 auf den Kapselsack 6 gedrückt wird, die zugehörige Schutzhülle 14 zu durchstoßen und sich in dem Kapselsack 6 zu verhaken. Die Adapter 2 sind an der Linsenhülle 1 befestigt und stehen von der Linsenhülle 1 nach innen ab. Ferner sind die Adapter 2 eingerichtet, in Eingriff mit jeweils einer Haptik 11 einer akkommodierenden Intraokularlinse 10 zu stehen.

Figuren 1 und 2 zeigen das in dem Kapselsack 6 angeordnete Augenimplantat 23 zu drei verschiedenen Zeitpunkten, wobei links der erste Zeitpunkt, in der Mitte der zweite Zeitpunkt und rechts der dritte Zeitpunkt dargestellt ist. Zu dem ersten Zeitpunkt ist der Hohlraum der Linsenhülle 1 mit einer Operationsflüssigkeit 7 gefüllt, die via das Durchgangsloch 18 in den Hohlraum eingebracht wurde. Zu dem zweiten Zeitpunkt wurde noch mehr von der Operationsflüssigkeit 7 in den Hohlraum eingebracht, wodurch die Linsenhülle 1 weiter als zu dem ersten Zeitpunkt gedehnt ist. Gemäß der ersten Ausführungsform werden dadurch die Reservoire 3 auf den Kapselsack 6 gedrückt, wodurch die Reservoire 3 den Klebstoff 4 abgeben und somit das Augenimplantat 23 mit dem Kapselsack 6 verklebt wird. Das mit dem Kapselsack 6 verklebte Augenimplantat 23 ist zu dem zweiten Zeitpunkt dargestellt. Indem die Linsenhülle 1 weiter gedehnt wird, wird gemäß der zweiten Ausführungsform der Widerhaken 13 auf den Kapselsack 6 gedrückt, wodurch der Widerhaken 13 die Schutzhülle 14 durchstößt und in dem Kapselsack 6 verhakt. Das mit den Widerhaken 13 in dem Kapselsack 6 verhakte Augenimplantat 23 ist zu dem zweiten Zeitpunkt dargestellt. Zu dem dritten Zeitpunkt ist dargestellt, dass in den Kapselsack 6 eine Einsetzöffnung 12 eingebracht werden kann, via die die akkommodierende Intraokularlinse 10 in den Hohlraum einbringbar ist. Es sind zusätzlich zu dem Kapselsack 6 der Ziliarmuskel 8 des Auges und die Zonulafasern 9 des Auges dargestellt. Es ist ein Zustand dargestellt, in dem der Ziliarmuskel 8 via die Zonulafasern 9 den Kapselsack 6 gestreckt hat und somit die akkommodierende Intraokularlinse 10 verformt hat. Es ist denkbar, dass die Reservoire 3 beziehungsweise die Widerhaken 13 alle in einer Ebene angeordnet sind. Dabei ist zudem denkbar, dass diese Ebene in dem Kapselsack 6 so angeordnet wird, dass sie in der Äquatorialebene der natürlichen Linse des Auges liegt.

Figur 1 zeigt, dass die Reservoire 3 jeweils eine Sollbruchstelle 5 haben können, die eingerichtet ist, wenn die Reservoire 3 auf den Kapselsack 6 gedrückt werden, geöffnet zu werden. Die Sollbruchstellen 5 können dabei so dimensioniert sein, dass sie bei einem ersten Druck reißen, wie er auftritt, wenn die Reservoire 3 auf den Kapselsack 6 gedrückt werden, jedoch bei einem zweiten Druck nicht reißen, der niedriger als der erste Druck ist und auftritt, wenn das Augenimplantat 23 durch eine Kanüle eines Injektors gedrückt wird. Der Fachmann kann Experimente durchführen, mit denen er ermitteln kann, wie die Sollbruchstellen 5 dazu dimensioniert werden müssen. Dazu kann er die Augenimplantate 23 mit verschieden dimensionierten Sollbruchstellen 5 herstellen und sie dem ersten Druck und dem zweiten Druck aussetzen.

Alternativ zu der Sollbruchstelle 5 ist denkbar, dass die Reservoire 3 jeweils eine Reservoiröffnung aufweisen, die eingerichtet ist, den Klebstoff 4 passieren zu lassen, wenn die Reservoire 3 auf den Kapselsack 6 gedrückt werden. Dazu können die Reservoiröffnungen beispielsweise so dimensioniert sein, dass sie, bevor sie auf den Kapselsack 6 gedrückt werden, so klein sind, dass kein Klebstoff 4 sie passieren kann, aber wenn sie auf den Kapselsack 6 gedrückt werden, die Reservoire 3 mitsamt den Reservoiröffnungen so gedehnt werden, dass der Klebstoff 4 die Reservoiröffnungen passieren kann.

Figur 1 zeigt, dass die Linsenhülle 1 für jede der Reservoire 3 eine Hüllenöffnung 27 aufweisen kann und an jeder der Hüllenöffnungen 27 einer der Adapter 2 angeordnet sein kann, der den Klebstoff 4 kontaktiert. Dabei ist es denkbar, dass der Adapter 2 so auf den Klebstoff 4 drückt, wenn der Hohlraum so von der Operationsflüssigkeit 7 gefüllt ist, dass die Reservoire 3 auf den Kapselsack 6 gedrückt werden, dass der Klebstoff 4 aus der Reservoiröffnung gedrückt wird oder die Sollbruchstelle 5 zum Reißen gebracht wird. Zudem ist aus Figur 1 ersichtlich, dass das Reservoir 3 sich durch die Hüllenöffnung 27 bis in das Innere der Linsenhülle 1 erstrecken kann. Der Adapter 2 kann somit besonders einfach mit dem Reservoir 3 verbunden werden, so dass kein Klebstoff 4 in das Innere der Linsenhülle 1 gelangen kann.

Figur 2 zeigt, dass jeder der Widerhaken 13 sich durch die Linsenhülle 1 erstrecken kann und an einem der Adapter 2 befestigt sein kann. Zudem kann der Widerhaken 13 eine Hakenspitze 24, einen Schaft 25 und einen Anker 26 aufweisen, wobei an einem Längsende des Schafts 25 die Hakenspitze 24 und an einem anderen Längsende des Schafts 25 der Anker 26 angeordnet ist, der an dem Adapter 2 befestigt sein kann. Der Anker 26 kann dabei in dem Inneren der Linsenhülle 1 angeordnet sein und die Hakenspitze 24 außerhalb der Linsenhülle 1 angeordnet sein. Dabei kann sich entweder der Anker 26 oder der Schaft 25 durch die Linsenhülle 1 erstrecken.

Wie es aus Figuren 3 oder 4 ersichtlich ist, kann die Linsenhülle 1 diskusförmig 15 oder torusförmig 16 sein, wobei jeweils links die diskusförmige 15 Ausführungsform und jeweils rechts die torusförmige 16 Ausführungsform dargestellt ist. Die torusförmige 16 Ausführungsform kann beispielsweise durch Rotation einer Fläche um eine Rotationsachse entstanden sein, wobei die Fläche vollständig in einer Ebene liegt, in der auch die Rotationsachse liegt. Beispielsweise ist denkbar, dass die Fläche die Form eines Kreises hat, wobei aber auch andere Formen, wie beispielsweise eine Ellipse, denkbar sind.

Figur 3 zeigt, dass die Linsenhülle 1 eine Linsenhüllenverdickung 17 aufweisen kann, die um das Durchgangsloch 18 herum angeordnet ist. Die Linsenhülle 1 kann auch ein Ventil aufweisen, welches das Durchgangsloch 18 aufweist. Hier kann die Linsenhüllenverdickung 17 ein Teil des Ventils sein. Dies gilt sowohl für die diskusförmige 15 Ausführungsform als auch für die torusförmige 16 Ausführungsform.

Wie es aus Figur 4 ersichtlich ist, kann die Linsenhülle 1 eine Hüllensollbruchstelle 19 aufweisen. Durch ein Öffnen der Hüllensollbruchstelle 19 kann die Einsetzöffnung 12 gebildet werden. Bei der Hüllensollbruchstelle 19 kann es sich beispielsweise um eine Perforierung handeln. Die Hüllensollbruchstelle 19 kann beispielsweise die Form einer geschlossenen Kurve haben, wie es auch für beide Ausführungsformen gemäß Figur 4 dargestellt ist. Dadurch kann bei der diskusförmigen 15 Ausführungsform die innerhalb der Sollbruchstelle 19 angeordnete Linsenhülle 1 aus dem Kapselsack 6 entfernt werden, wodurch die Menge an Material, die in dem Kapselsack 6 verbleibt, verringert werden. In dem Fall, dass die Linsenhüllenverdickung 17 vorgesehen ist, ist es somit vorteilhaft, die Linsenhüllenverdickung 17 innerhalb der Sollbruchstelle 19 vorzusehen, um besonders viel Material aus dem Kapselsack 6 zu entfernen. Bei der torusförmigen 16 Ausführungsform kann die Hüllensollbruchstelle 19 an einer Stirnseite der Linsenhülle 1 vorgesehen sein, wie es auch in Figur 4 dargestellt ist. Alternativ ist denkbar, dass die Hüllensollbruchstelle 19 an der Innenseite der Linsenhülle 1 vorgesehen ist. Alternativ ist denkbar, dass die Sollbruchstelle 19 die Form einer Linie hat, die einen Anfangspunkt und einen Endpunkt hat und die gerade und/oder gekrümmt sein kann.

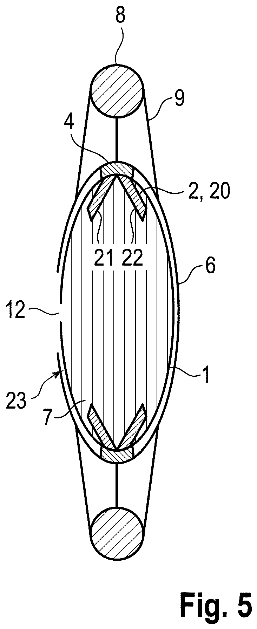

Figur 5 zeigt, dass jeder der Adapter 2 als ein offener Adapter 20 ausgeführt sein kann und einen ersten Adapterarm 21 und einen zweiten Adapterarm 22 aufweisen kann, die eingerichtet sind, dass zwischen ihnen die Haptik 11 in Eingriff mit ersten Adapterarm 21 und dem zweiten Adapterarm 22 steht. Der erste Adapterarm 21 und der zweite Adapterarm 22 sind V-förmig angeordnet, wobei der Abstand zwischen dem ersten Adapterarm 21 und dem zweiten Adapterarm 22 immer länger wird, je weiter der erste Adapterarm 21 und der zweite Adapterarm 22 nach innen ragen.

Die Operationsflüssigkeit 7 kann ein Gleitmittel und/oder eine physiologische Salzlösung aufweisen oder aus dem Gleitmittel und/oder der physiologischen Salzlösung bestehen. Bei dem Gleitmittel kann es sich um ein sogenanntes opthalmisches Viskoelastikum handeln (engl.: opthalmic viscoelastic device (OVD)).

### Bezugszeichenliste

- 1: Linsenhülle
- 2: Adapter
- 3: Reservoir
- 4: Klebstoff
- 5: Sollbruchstelle
- 6: Kapselsack
- 7: Operationsflüssigkeit
- 8: Ziliarmuskel
- 9: Zonulafasern
- 10: akkommodierende Intraokularlinse
- 11: Haptik
- 12: Einsetzöffnung
- 13: Widerhaken
- 14: Schutzhülle
- 15: Kugel
- 16: Torus
- 17: Linsenhüllenverdickung
- 18: Durchgangsloch
- 19: Hüllensollbruchstelle
- 20: offener Adapter
- 21: erster Adapterarm
- 22: zweiter Adapterarm
- 23: Augenimplantat
- 24: Hakenspitze
- 25: Schaft
- 26: Anker
- 27: Hüllenöffnung

## Patentansprüche

1. Augenimplantat mit einer Linsenhülle (1), die eingerichtet ist, in einen Kapselsack (6) eines Auges eingesetzt zu werden, elastisch ist, in ihrem Inneren einen Hohlraum begrenzt und ein Durchgangsloch (18) aufweist, via das von außerhalb der Linsenhülle (1) der Hohlraum zugänglich ist, sowie diskusförmig oder torusförmig ist, und mindestens zwei Adaptern (2), die an der Linsenhülle (1) befestigt sind, von der Linsenhülle (1) nach innen abstehen und eingerichtet sind, in Eingriff mit jeweils einer Haptik (11) einer akkommodierenden Intraokularlinse (10) zu stehen, **dadurch gekennzeichnet, dass** das Augenimplantat (23) eine Mehrzahl an Reservoiren (3) aufweist, die an der Linsenhülle (1) befestigt sind, von der Linsenhülle (1) nach außen abstehen, in ihrem Inneren einen Klebstoff (4) aufweisen und eingerichtet sind, wenn sie auf den Kapselsack (6) gedrückt werden, den Klebstoff (4) abzugeben.

2. Augenimplantat gemäß Anspruch **1,** wobei die Reservoire (3) jeweils eine Sollbruchstelle (5) haben, die eingerichtet ist, geöffnet zu werden, wenn die Reservoire (3) auf den Kapselsack (6) gedrückt werden, oder wobei die Reservoire (3) jeweils eine Reservoiröffnung aufweisen, die eingerichtet ist, den Klebstoff (4) passieren zu lassen, wenn die Reservoire (3) auf den Kapselsack (6) gedrückt werden.

3. Augenimplantat gemäß Anspruch 1 oder 2, wobei die Linsenhülle (1) für jede der Reservoire (3) eine Hüllenöffnung (27) aufweist und an jeder der Hüllenöffnungen (27) einer der Adapter (2) angeordnet ist, der den Klebstoff (4) kontaktiert.

4. Augenimplantat mit einer Linsenhülle (1), die eingerichtet ist, in einen Kapselsack (6) eines Auges eingesetzt zu werden, elastisch ist, in ihrem Inneren einen Hohlraum begrenzt und ein Durchgangsloch (18) aufweist, via das von außerhalb der Linsenhülle (1) der Hohlraum zugänglich ist, sowie diskusförmig oder torusförmig ist, und mindestens zwei Adaptern (2), die an der Linsenhülle (1) befestigt sind, von der Linsenhülle (1) nach innen abstehen und eingerichtet sind, in Eingriff mit jeweils einer Haptik (11) einer akkommodierenden Intraokularlinse (10) zu stehen, **dadurch gekennzeichnet, dass** das Augenimplantat (23) eine Mehrzahl an Schutzhüllen (14), die an der Linsenhülle (1) befestigt sind und von der Linsenhülle (1) nach außen abstehen, und jeweils einen Widerhaken (13) für jede Schutzhülle (14) aufweist, der in dem Inneren der zugehörigen Schutzhülle (14) eingekapselt ist, mit der Linsenhülle (1) verbunden ist, von der Linsenhülle (1) nach außen absteht und eingerichtet ist, wenn der Widerhaken (13) auf den Kapselsack (6) gedrückt wird, die zugehörige Schutzhülle (14) zu durchstoßen und in dem Kapselsack (6) zu verhaken.

5. Augenimplantat gemäß Anspruch 4, wobei jeder der Widerhaken (13) sich durch die Linsenhülle (1) erstreckt und an einem der Adapter (2) befestigt ist.

6. Augenimplantat gemäß Anspruch 4 oder 5, wobei die Linsenhülle (1) diskusförmig (15) oder torusförmig (16) ist.

7. Augenimplantat gemäß einem der Ansprüche 1 bis 6, wobei die Linsenhülle (1) eine Linsenhüllenverdickung (17), die um das Durchgangsloch (18) herum angeordnet ist, und/oder ein Ventil aufweist, das das Durchgangsloch (18) aufweist.

8. Augenimplantat gemäß einem der Ansprüche 1 bis 7, wobei die Linsenhülle (1) eine Hüllensollbruchstelle (19) aufweist.

9. Augenimplantat gemäß einem der Ansprüche 1 bis 8, wobei jeder der Adapter (2) einen ersten Adapterarm (21) und einen zweiten Adapterarm (22) aufweist, die eingerichtet sind, dass zwischen ihnen die Haptik (11) in Eingriff steht.

10. Augenimplantat gemäß einem der Ansprüche 1 bis 9, wobei das Augenimplantat (23) mindestens zwei Magneten aufweist, die eingerichtet sind, jeweils eine der Haptiken (11) an dem Augenimplantat (23) zu befestigen.

## Claims

1. Eye implant having a lens sheath (1) which is configured to be inserted into a capsular bag (6) of an eye, which is elastic, which delimits a cavity in its interior and which has a through hole (18), via which the cavity is accessible from outside the lens sheath (1), and which is discus-shaped or toroidal, and having at least two adapters (2) which are secured to the lens sheath (1), which project inwardly from the lens sheath (1) and which are configured to engage with a respective haptic (11) of an accommodating intraocular lens (10), **characterized in that** the eye implant (23) comprises a plurality of reservoirs (3) which are secured to the lens sheath (1), which project outwardly from the lens sheath (1), which comprise an adhesive (4) in their interior and which are configured to release the adhesive (4) when pressed against the capsular bag (6).

2. Eye implant according to Claim 1, wherein the reservoirs (3) each have a predetermined breaking point (5) which is configured to be opened when the reservoirs (3) are pressed against the capsular bag (6), or wherein the reservoirs (3) each have a reservoir opening which is configured to allow the adhesive (4) to pass when the reservoirs (3) are pressed against the capsular bag (6).

3. Eye implant according to Claim 1 or 2, wherein the lens sheath (1) has a sheath opening (27) for each of the reservoirs (3) and one of the adapters (2) which is in contact with the adhesive (4) is arranged at each of the sheath openings (27).

4. Eye implant having a lens sheath (1) which is configured to be inserted into a capsular bag (6) of an eye, which is elastic, which delimits a cavity in its interior and which has a through hole (18), via which the cavity is accessible from outside the lens sheath (1), and which is discus-shaped or toroidal, and having at least two adapters (2) which are secured to the lens sheath (1), which project inwardly from the lens sheath (1) and which are configured to engage with a respective haptic (11) of an accommodating intraocular lens (10), **characterized in that** the eye implant (23) comprises a plurality of protective sheaths (14) which are secured to the lens sheath (1) and project outwardly from the lens sheath (1) and comprises a respective barb (13) for each protective sheath (14), said barb being encapsulated in the interior of the associated protective sheath (14), being connected to the lens sheath (1), protruding outwardly from the lens sheath (1) and being configured to pierce the associated protective sheath (14) and hook into the capsular bag (6) when said barb (13) is pressed against the capsular bag (6).

5. Eye implant according to Claim 4, wherein each of the barbs (13) extends through the lens sheath (1) and is secured to one of the adapters (2).

6. Eye implant according to Claim 4 or 5, wherein the lens sheath (1) is discus-shaped (15) or toroidal (16).

7. Eye implant according to any of Claims 1 to 6, wherein the lens sheath (1) has a lens sheath swelling (17) arranged around the through hole (18) and/or comprises a valve which contains the through hole (18).

8. Eye implant according to any of Claims 1 to 7, wherein the lens sheath (1) has a predetermined sheath breaking point (19).

9. Eye implant according to any of Claims 1 to 8, wherein each of the adapters (2) comprises a first adapter arm (21) and a second adapter arm (22) which are configured such that the haptic (11) engages therebetween.

10. Eye implant according to any of Claims 1 to 9, wherein the eye implant (23) comprises at least two magnets which are configured to each secure one of the haptics (11) to the eye implant (23).

## Revendications

1. Implant oculaire comprenant une enveloppe de cristallin (1) qui est adaptée pour être insérée dans un sac capsulaire (6) d'un œil, qui est élastique, qui délimite une cavité à l'intérieur d'elle-même et qui présente un trou traversant (18) via lequel la cavité est accessible depuis l'extérieur de l'enveloppe de cristallin (1), et qui est en forme de disque ou de tore, et au moins deux adaptateurs (2) qui sont fixés à l'enveloppe de cristallin (1), qui font saillie vers l'intérieur depuis l'enveloppe de cristallin (1) et qui sont adaptés pour venir en engagement avec une haptique (11) respective d'un cristallin intraoculaire accommodant (10), **caractérisé en ce que** l'implant oculaire (23) comprend une pluralité de réservoirs (3) qui sont fixés à l'enveloppe de cristallin (1), font saillie vers l'extérieur à partir de l'enveloppe de cristallin (1), contiennent un adhésif (4) à l'intérieur et sont adaptés, lorsqu'ils sont pressés sur le sac capsulaire (6), pour libérer l'adhésif (4).

2. Implant oculaire selon la revendication 1, dans lequel les réservoirs (3) ont chacun un point destiné à la rupture (5) qui est conçu pour être ouvert lorsque les réservoirs (3) sont pressés sur le sac capsulaire (6), ou dans lequel les réservoirs (3) présentent chacun une ouverture de réservoir qui est conçue pour laisser passer la colle (4) lorsque les réservoirs (3) sont pressés sur le sac capsulaire (6).

3. Implant oculaire selon la revendication 1 ou la revendication 2, dans lequel l'enveloppe de cristallin (1) présente une ouverture d'enveloppe (27) pour chacun des réservoirs (3) et l'un des adaptateurs (2) est agencé sur chacune des ouvertures d'enveloppe (27), lequel est en contact avec la colle (4).

4. Implant oculaire comprenant une enveloppe de cristallin (1) qui est conçue pour être insérée dans un sac capsulaire (6) d'un œil, qui est élastique, qui délimite une cavité à l'intérieur d'elle-même et qui présente un trou traversant (18) via lequel la cavité est accessible depuis l'extérieur de l'enveloppe de cristallin (1), et est en forme de disque ou de tore, et au moins deux adaptateurs (2) qui sont fixés à l'enveloppe de cristallin (1), font saillie vers l'intérieur à partir de l'enveloppe de cristallin (1) et sont adaptés pour venir chacun en engagement avec une haptique (11) d'un cristallin intraoculaire accommodant (10), **caractérisé en ce que** l'implant oculaire (23) comprend une pluralité d'enveloppes de protection (14) fixées à l'enveloppe de cristallin (1) et faisant saillie vers l'extérieur à partir de l'enveloppe de cristallin (1), et un barbillon respectif (13) pour chaque enveloppe de protection (14), encapsulé à l'intérieur de l'enveloppe de protection (14) associée, est relié à l'enveloppe de cristallin (1), fait saillie vers l'extérieur de l'enveloppe de cristallin (1) et est conçu, lorsque le barbillon (13) est pressé sur le sac capsulaire (6), pour percer l'enveloppe de protection (14) correspondante et s'accrocher dans le sac capsulaire (6) .

5. Implant oculaire selon la revendication 4, dans lequel chacun des barbillons (13) s'étend à travers l'enveloppe (1) du cristallin et est fixé à l'un des adaptateurs (2).

6. Implant oculaire selon la revendication 4 ou la revendication 5, dans lequel l'enveloppe de cristallin (1) est en forme de disque (15) ou de tore (16).

7. Implant oculaire selon l'une des revendications 1 à 6, dans lequel l'enveloppe de cristallin (1) comprend un épaississement (17) d'enveloppe de cristallin agencé autour du trou traversant (18) et/ou une vanne comprenant le trou traversant (18).

8. Implant oculaire selon l'une des revendications 1 à 7, dans lequel l'enveloppe de cristallin (1) présente une zone de rupture de l'enveloppe (19).

9. Implant oculaire selon l'une des revendications 1 à 8, dans lequel chacun des adaptateurs (2) comprend un premier bras d'adaptateur (21) et un deuxième bras d'adaptateur (22) agencés de façon que l'haptique (11) soit engagée entre eux.

10. Implant oculaire selon l'une des revendications 1 à 9, dans lequel l'implant oculaire (23) comprend au moins deux aimants agencés pour fixer chacune des haptiques (11) à l'implant oculaire (23).
